# EUROPÄISCHE PATENTSCHRIFT

(11) **EP 0 498 847 B1**
(45) Veröffentlichungstag und Bekanntmachung des Hinweises auf die Patenterteilung: **14.09.1994**
(21) Anmeldenummer: 90917093.8
(22) Anmeldetag: 25.10.1990
(51) Int. Cl.: C07C 49/83, C07C 45/54

(54) **VERFAHREN ZUR HERSTELLUNG VON 1,4-BIS-(4-HYDROXYBENZOYL)-BENZOL**
PROCESS FOR PRODUCING 1,4-bis-(4-HYDROXYBENZOYL)BENZOL
PROCEDE DE PREPARATION DE 1,4-BIS-(4-HYDROXYBENZOYL)-BENZOL

(30) Priorität: 02.11.1989 DE 3936399
(43) Veröffentlichungstag der Anmeldung: 19.08.1992
(73) Patentinhaber: HOECHST AKTIENGESELLSCHAFT, 65926 Frankfurt am Main (DE)
(72) Erfinder: HACKENBRUCH, Joachim, Dr., D-6500 Mainz (DE); PAPENFUHS, Theodor, D-6000 Frankfurt am Main (DE); SCHNELLER, Arnold, D-6500 Mainz (DE)
(86) Internationale Anmeldenummer: EP9001809
(87) Internationale Veröffentlichungsnummer: WO9106524

(56) Entgegenhaltungen:
- EP-A- 0 057 503
- EP-A- 0 232 992
- JOURNAL OF THE AMERICAN CHEMICAL SOCIETY, Band 60, Nr. 10, 8. Oktober 1938; F. F. BLICKE et al: "The action of aluminum chloride on the diphenyl ester of isophthalic, terephthalic and naphthalic acids", S. 2283-2285
- CHEMICAL ABSTRACTS, Band 109, Nr. 11, September 1988, Columbus, Ohio, USA; S. FUKUOKA et al, "Preparation of p-(p-fluorobenzoyl)phenol derivatives as pharmaceutical and agrochemical intermediates or polymer materials", Seite 658, Zusammenfassung 92486h; & JP-A-63 23 831

## Beschreibung

Die Erfindung betrifft ein Verfahren zur Herstellung von 1,4-Bis-(4-hydroxybenzoyl)benzol durch Fries'sche Umlagerung von Terephthalsäurediphenylester in Gegenwart von Halogenalkansulfonsäure in Lösungsmitteln.

Blicke et al. beschreiben in J.A.C.S. 1938, S. 2283 die Umlagerung von Terephthalsäurediphenylester in Gegenwart von AlCl₃ in Schwefelkohlenstoff (CS₂). Nach Abdestillation des Lösungsmittels wird das Reaktionsgemisch auf 185-190°C erhitzt. Nach anschließender Zerstörung des Aluminiumkomplexes mit verdünnter Salzsäure wird das erhaltene Produkt mit verdünnter wäßriger Natronlauge extrahiert. Abschließendes Ansäuern ergibt dann das 1,4-Bis-(4-hydroxybenzoyl)benzol, das nach Umkristallisation aus Alkohol einen Schmelzpunkt von 297-299°C zeigt. Dieser Syntheseweg ist aber für eine technische Herstellung wegen damit verbundener Nachteile, wie aluminiumhaltiges Abwasser und eine Vielzahl von Nebenreaktionen durch die beträchtlich hohe Temperatur, nicht geeignet.

Aus der EP 0075390 ist ein Verfahren zur Herstellung von 1,4-Bis-(4-hydroxybenzoyl)benzol bekannt, wobei, ausgehend von Phenol und Terephthalsäure in Fluoralkansulfonsäure als Lösungsmittel, die Zielverbindung hergestellt wird.

Es bestand daher ein Bedürfnis nach einem Verfahren, bei welchem das Katalysatorsystem in unterstöchiometrischen Mengen verwendet, leicht aufgearbeitet und zurückgewonnen werden kann.

Es wurde nun gefunden, daß man 1,4-Bis-(4-hydroxybenzoyl)benzol der Formel I 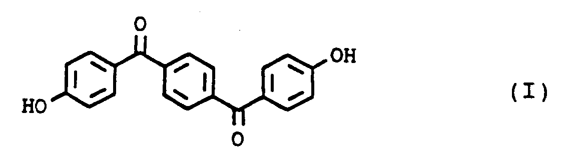
vorteilhaft herstellen kann, indem man Terephthalsäurediphenylester der Formel II 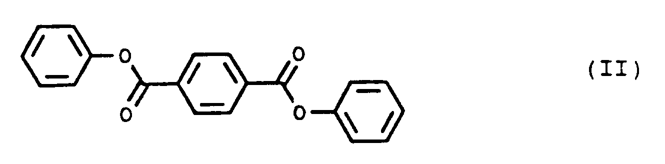
in 3 bis 50 Teilen, vorzugsweise 5 bis 20 Teilen, eines gegenüber den Reaktionspartnern inerten wasserfreien organischen Lösungsmittels, bezogen auf den Terephthalsäurediphenylester, in Gegenwart von 30 bis 500 Mol-% Halogenalkansulfonsäuren (Katalysatoren) der allgemeinen Formel III

Y(CₙX₂ₙ)SO₃H (III),

vorzugsweise der allgemeinen Formel (IV)

Y(CₙF₂ₙ)SO₃H (IV),

worin Y ein Fluor- oder Wasserstoffatom darstellt,
X ein Fluor- und/oder Chloratom bedeutet,
mit der Maßgabe, daß mindestens ein X für ein Fluoratom steht, und n eine ganze Zahl von 1 bis 10 bedeutet, bei Temperaturen von 10°C bis 200°C, vorzugsweise von etwa 80°C bis etwa 130°C, umlagert.

Es ist beim erfindungsgemäßen Verfahren vorteilhaft, unter sauerstoffreien Bedingungen zu arbeiten. Was die Druckverhältnisse anbelangt, so kann man bei Atmosphärendruck oder auch bei leichtem Überdruck, insbesondere beim Eigendruck des Systems, arbeiten, der insbesondere durch die angewandte Temperatur, aber auch durch andere Faktoren, wie das Ausmaß der Füllung des Reaktionsgefäßes bestimmt wird. Beispielsweise kann der Überdruck im Bereich bis zu 10 oder 15 bar liegen. Wenn man bei erhöhtem Druck arbeitet, kann man das Verfahren beispielsweise in einem Autoklav aus Edelstahl, der gegebenenfalls mit einem resistenten Material, wie beispielsweise Polytetrafluorethylen, ausgekleidet ist, durchführen. Durch die Anwendung von Überdruck kann die Reaktion beschleunigt werden, jedoch kann damit auch eine erhöhte Bildung von Nebenprodukten verbunden sein.

Ein Vorteil des erfindungsgemäßen Verfahrens besteht darin, daß die nach der Abtrennung des ausgefallenen 1,4-Bis-(4-hydroxybenzoyl)benzols erhaltene Mutterlauge wieder mit Terephthalsäurediphenylester oder Phenol/Terephthalsäuredichlorid versetzt wird und so das Reaktionsmedium vielfach, z.B. bis zu sechsmal wiederverwendet werden kann, ohne daß merkliche Ausbeuteverluste eintreten. Bei zu häufiger Rückführung wird eine Anreicherung von Nebenprodukten beobachtet, so daß es sich als günstig erweist, ca. 20 Gew.-% auszuschleusen und mit frischem Katalysator aufzustärken. Die ausgeschleuste Mutterlauge kann nach bekannten Verfahren extern aufgearbeitet werden, um den Katalysator zurückzugewinnen. Eine zweite Möglichkeit der Aufarbeitung ist die Aufkonzentrierung des Reaktionsgemisches durch Abdestillieren des Lösungsmittel-/Katalysatorgemisches, das ebenso in die nächste Umsetzung zurückgeführt werden kann. Welche der beiden Methoden sich am günstigsten erweist, hängt vom angewandten Lösungsmittel und der eingesetzten Halogenalkansulfonsäure ab. Das abgetrennte rohe 1,4-Bis-(4-hydroxybenzoyl)benzol kann durch Behandlung mit Alkalihydroxiden und nachfolgende pH-gesteuerte Ansäuerung aufgereinigt und vom mitgebildeten Halbester abgetrennt werden, welcher in die nächste Umsetzung zurückzuführen ist.

Die erfindungsgemäß verwendeten Katalysatoren der genannten Formel (III) enthalten vorzugsweise nicht mehr als 3 Chloratome, insbesondere höchstensein Chloratom. Die Gruppe CₙX₂ₙ bzw. CₙF₂ₙ kann geradkettig oder verzweigt sein, wobei Verbindungen, in welcher Y ein Wasserstoffatom ist, dieses vielfach in β-Stellung enthalten.

Die erfindungsgemäß verwendeten perfluorierten Katalysatoren sind bekannt (vgl. DE-OS 21 39 994), ebenso die β-H-Perfluoralkansulfonsäuren (J. Am. Chem. Soc. 75, 44595-44596, 1953). Soweit sie noch nicht bekannt sind, können sie nach den üblichen Verfahren erhalten werden. Geeignete Verbindungen der genannten allgemeinen Formel (III) sind beispielsweise Perfluor-n-octansulfonsäure, Perfluorhexansulfonsäure, Perfluorbutansulfonsäure, Pentafluorethansulfonsäure, β-H-Perfluorheptansulfonsäure und β-H-Perfluorpentansulfonsäure. Es können auch Mischungen verschiedener Katalysatoren verwendet werden. Bevorzugt sind Trifluormethansulfonsäure, 2-Chlor-1,1,2-trifluorethansulfonsäure und 2-Hydroperfluorpropansulfonsäure.

Im allgemeinen setzt man den Katalysator im ersten Ansatz in Mengen von etwa 30 bis etwa 500 Mol-%, bezogen auf den Terephthalsäurediphenylester, zu. Verwendet man die Mutterlauge nach Abtrennung des erwünschten Reaktionsproduktes oder das abdestillierte Lösungsmittel-/Katalysatorgemisch wieder, indem man sie zurückführt, wird als Ergebnis in der Regel eine unterstöchiometrische Menge an Katalysator verwendet.

Als inerte organische Lösungsmittel können beispielsweise Chlorbenzol, o-Dichlorbenzol, m-Dichlorbenzol, o-Chlortoluol, Nitrobenzol, N-Methylpyrrolidon oder Sulfolan eingesetzt werden.

Man kann die Umlagerung auch in einem Eintopfverfahren, ausgehend von Phenol und Terephthalsäuredichlorid, ohne Zwischenisolierung des Terephthalsäurediphenylesters, vornehmen, wobei die vorausgehene Veresterung bei Temperaturen von etwa 10 bis etwa 200°C vorgenommen wird. Auch bei dieser Verfahrensweise kann man das nach beendeter Umlagerung angefallene Reaktionsgemisch nach Abtrennung des ausgefallenen 1,4-Bis-(4-hydroxybenzoyl)benzols mit Phenol/Terephthalsäuredichlorid versetzen und wiederverwenden.

Das nach dem erfindungsgemäßen Verfahren erhaltene 1,4-Bis-(4-hydroxybenzoyl)benzol der genannten Formel (I) ist ein wichtiges Monomer für chemikalienbeständige Kunststoffe, die auch bei hohen Temperaturen noch beständig sind. Die Verbindung der Formel (I) kann, sofern erforderlich, aus Lösungsmitteln, wie beispielsweise Ethanol, umkristallisiert werden.

Die Erfindung wird durch die nachstehenden Beispiele erläutert, ohne darauf beschränkt zu werden. In diesen Beispielen bedeuten h Stunde und BHB 1,4-Bis-(4-hydroxybenzoyl)benzol.

### Beispiele

1.) 0,25 Mol Terephthalsäurediphenylester und 0,25 Mol Hexafluorpropansulfonsäure werden in 300 ml Chlorbenzol für 10 h auf 100-110°C erhitzt. Nach dem Kaltrühren wird das ausgefallene BHB abfiltriert, mit 50 ml Chlorbenzol nachgewaschen und das Rohprodukt getrocknet. Die Mutterlauge kann in die nächste Umsetzung zurückgeführt werden.

| | |
|---|---|
| Ausbeute: | 85 % d.Th. |
| Reinheit (HPLC): | 92 % |
| Fp.: | 312 - 315°C. |

Verwendet man anstelle der 300 ml Chlorbenzol die gleiche Menge o-Dichlorbenzol, m-Dichlorbenzol oder o-Chlortoluol und arbeitet im übrigen wie in diesem Beispiel angegeben, so gelangt man praktisch zum gleichen Ergebnis.
Verwendet man anstelle von 0,25 Mol Hexafluorpropansulfonsäure die gleiche Molmenge Trifluormethansulfonsäure, Perfluorbutansulfonsäure oder 2-Chlor-1,1,2-trifluorethansulfonsäure und arbeitet im übrigen wie in diesem Beispiel angegeben, so erreicht man praktisch das gleiche Resultat.
2.) 0,5 Mol Phenol, 0,25 Mol Terephthalsäuredichlorid und 0,50 Mol Hexafluorpropansulfonsäure werden in 500 ml Nitrobenzol bei Raumtemperatur vorgelegt und für 3 h unter Salzsäureentwicklung bei der angegebenen Temperatur gerührt. Nach beendeter Gasentwicklung wird auf 80°C erhitzt und innerhalb von 2 h auf 100°C hochgeheizt und 8 h lang gerührt. Nach dem Kaltrühren wird vom ausgefallenen BHB abfiltriert, mit 50 ml Nitrobenzol nachgewaschen und das Rohprodukt getrocknet. Die Mutterlauge kann in die nächste Umsetzung zurückgeführt werden.

| | |
|---|---|
| Ausbeute: | 80 - 85 % d.Th. |
| Reinheit (HPLC): | 90 - 92 % |
| Fp.: | 312 - 314°C. |

3.) 0,5 Mol Phenol, 0,25 Mol Terephthalsäuredichlorid und 0,5 Mol Trifluormethansulfonsäure werden in 500 ml Nitrobenzol bei Raumtemperatur vorgelegt und 3 h lang unter Salzsäureentwicklung bei der angegebenen Temperatur gerührt. Nach beendeter Gasentwicklung wird auf 80°C erhitzt und weitere 10 h gerührt. Anschließend wird das Reaktionsgemisch im Feinvakuum (0,1 mbar) bei 100°C auf ein Fünftel des Volumens aufkonzentriert. Das anfallende Destillat kann für die nächste Umsetzung wiederverwendet werden. Den Rückstand verdünnt man mit 200 ml Aceton, rührt ihn in 1 l Wasser ein und filtriert das dabei ausfallende Rohprodukt ab.

Die Aufreinigung wird folgendermaßen vorgenommen:
Der Feststoff wird mit 200 ml Natriumhydrogencarbonat (10 %ig) gewaschen und in 2,5 l einer 0,2 n K₂CO₃-Lösung gelöst. Durch langsames Ansäuern der Lösung mit Salzsäure auf pH 8,5 wird der als Nebenprodukt entstandene Halbester ausgefällt und durch Filtration abgetrennt. Durch weiteres Ansäuern auf pH 6 wird das Produkt ausgefällt.

| | |
|---|---|
| Ausbeute: | 75 % d.Th. |
| Reinheit (HPLC): | 96 % |
| Fp.: | 317 - 318°C. |

## Patentansprüche

1. Verfahren zur Herstellung von 1,4-Bis-(4-hydroxybenzoyl)benzol der Formel (I) 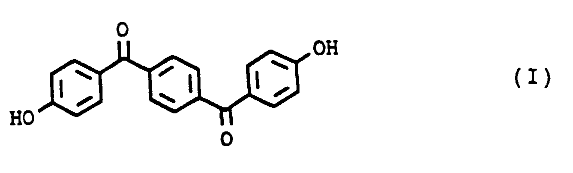 dadurch gekennzeichnet, daß man Terephthalsäurediphenylester der Formel (II) 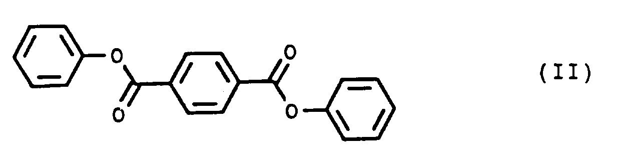 in 3 bis 50 Teilen eines gegenüber den Reaktionspartnern inerten wasserfreien organischen Lösungsmittels, bezogen auf den Terephthalsäurediphenylester, in Gegenwart von 30 bis 500 Mol-% einer Halogenalkansulfonsäure (Katalysator) der allgemeinen Formel (III) oder (IV)
Y(CₙX₂ₙ)SO₃H (III)
Y(CₙF₂ₙ)SO₃H (IV),
worin Y ein Fluor- oder Wasserstoffatom bedeutet, X für ein Fluor- und/oder Chloratom steht, mit der Maßgabe, daß mindestens ein X für ein Fluoratom steht, und n eine ganze Zahl von 1 bis 10 bedeutet, bei Temperaturen von 10 bis 200°C umlagert.

2. Verfahren nach Anspruch 1, dadurch gekennzeichnet, daß man bei Temperaturen von etwa 50°C bis etwa 160°C umlagert.

3. Verfahren nach Anspruch 1, dadurch gekennzeichnet, daß man bei Temperaturen von etwa 80°C bis etwa 130°C umlagert.

4. Verfahren nach mindestens einem der Ansprüche 1 bis 3, dadurch gekennzeichnet, daß Y in den allgemeinen Formeln (III) oder (IV) ein Wasserstoffatom bedeutet und dieses in β-Stellung zur -SO₃H-Gruppe steht.

5. Verfahren nach mindestens einem der Ansprüche 1 bis 4, dadurch gekennzeichnet, daß man in Gegenwart von Hexafluorpropansulfonsäure oder Trifluormethansulfonsäure umlagert.

6. Verfahren nach mindestens einem der Ansprüche 1 bis 5, dadurch gekennzeichnet, daß man in Chlorbenzol, Dichlorbenzol, Chlortoluol, Nitrobenzol, N-Methylpyrrolidon oder Sulfolan als inertem organischem Lösungsmittel umlagert.

7. Verfahren nach mindestens einem der Ansprüche 1 bis 6, dadurch gekennzeichnet, daß man in etwa 5 bis etwa 20 Teilen des inerten organischen Lösungsmittels, bezogen auf den Terephthalsäurediphenylester, umlagert.

8. Verfahren nach mindestens einem der Ansprüche 1 bis 7, dadurch gekennzeichnet, daß man die Umlagerung des Terephtahlsäurediphenylesters im Druckbereich Atmosphärendruck bis zum Eigendruck des Reaktionsgemisches unter den angewandten Reaktionsbedingungen durchführt.

9. Verfahren nach mindestens einem der Ansprüche 1 bis 8, dadurch gekennzeichnet, daß man im Druckbereich Atmosphärendruck bis etwa 15 bar umlagert.

10. Verfahren nach mindestens einem der Ansprüche 1 bis 9, dadurch gekennzeichnet, daß man in Abwesenheit von Sauerstoff umlagert.

11. Verfahren nach mindestens einem der Ansprüche 1 bis 10, dadurch gekennzeichnet, daß man die angefallene Mutterlauge nach Abtrennung des 1,4-Bis-(4-hydroxybenzoyl)benzols wiederverwendet.

12. Verfahren nach mindestens einem der Ansprüche 1 bis 11, dadurch gekennzeichnet, daß man das nach beendeter Umlagerung angefallene Reaktionsgemisch nach Abtrennung des 1,4-Bis-(4-hydroxybenzoyl)benzols mit Terephthalsäurediphenylester versetzt und wiederverwendet.

13. Verfahren nach mindestens einem der Ansprüche 1 bis 10, dadurch gekennzeichnet, daß man die Umlagerung in einem Eintopfverfahren, ausgehend von Phenol und Terephthalsäuredichlorid, ohne Zwischenisolierung des Terephthalsäurediphenylesters, vornimmt, wobei die vorausgehende Veresterung bei Temperaturen von etwa 10 bis etwa 200°C durchgeführt wird.

14. Verfahren nach Anspruch 13, dadurch gekennzeichnet, daß man das nach beendeter Umlagerung angefallene Reaktionsgemisch nach Abtrennung des 1,4-Bis-(4-hydroxybenzoyl)benzols mit Phenol/Terephthalsäuredichlorid versetzt und wiederverwendet.

## Claims

1. A process for the preparation of 1,4-bis(4-hydroxybenzoyl)benzene of the formula (I) 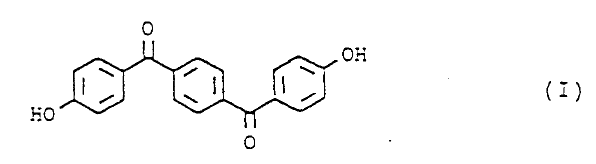 which comprises rearranging diphenyl terephthalate of the formula (II) 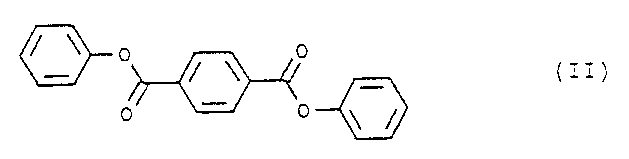 in 3 to 50 parts, relative to the diphenyl terephthalate, of an anhydrous organic solvent which is inert to the reactants, in the presence of 30 to 500 mol-% of a haloalkanesulfonic acid (catalyst) of the general formula (III) or (IV)
Y(CₙX₂ₙ)SO₃H (III)
Y(CₙF₂ₙ)SO₃H (IV),
in which Y is a fluorine or hydrogen atom, and in which Y is a fluorine or hydrogen atom, and X is a fluorine and/or chlorine atom, with the proviso that at least one X is a fluorine atom, and n is an integer from 1 to 10, at temperatures of 10 to 200°C.

2. The process as claimed in claim 1, wherein the rearrangement is carried out at temperatures of about 50°C to about 160°C.

3. The process as claimed in claim 1, wherein the rearrangement is carried out at temperatures of about 80°C to about 130°C.

4. The process as claimed in at least one of claims 1 to 3, wherein Y in the general formulae (III) or (IV) is a hydrogen atom and this is in the β-position to the -SO₃H group.

5. The process as claimed in at least one of claims 1 to 4, wherein the rearrangement is carried out in the presence of hexafluoropropanesulfonic acid or trifluoromethanesulfonic acid.

6. The process as claimed in at least one of claims 1 to 5, wherein the rearrangement is carried out in chlorobenzene, dichlorobenzene, chlorotoluene, nitrobenzene, N-methylpyrrolidone or sulfolane as inert organic solvent.

7. The process as claimed in at least one of claims 1 to 6, wherein the rearrangement is carried out in about 5 to about 20 parts of the inert organic solvent, relative to the diphenyl terephthalate.

8. The process as claimed in at least one of claims 1 to 7, wherein the rearrangement of the diphenyl terephthalate is carried out in the pressure range from atmospheric pressure up to the autogenous pressure of the reaction mixture under the reaction conditions used.

9. The process as claimed in at least one of claims 1 to 8, wherein the rearrangement is carried out in the pressure range from atmospheric pressure up to about 15 bar.

10. The process as claimed in at least one of claims 1 to 9, wherein the rearrangement is carried out in the absence of oxygen.

11. The process as claimed in at least one of claims 1 to 10, wherein the mother liquor obtained after separating off the 1,4-bis(4-hydroxybenzoyl)benzene is reused.

12. The process as claimed in at least one of claims 1 to 11, wherein diphenyl terephthalate is added to the reaction mixture obtained after completion of the rearrangement after separating off the 1,4-bis(4-hydroxybenzoyl)benzene and the mixture is reused.

13. The process as claimed in at least one of claims 1 to 10, wherein the rearrangement is carried out in a one-pot process, starting from phenol and terephthaloyl dichloride, without intermediate isolation of the diphenyl terephthalate, the initial esterification being carried out at temperatures of about 10 to about 200°C.

14. The process as claimed in claim 13, wherein phenol/terephthaloyl dichloride is added to the reaction mixture obtained after completion of the rearrangement after separating off the 1,4-bis(4-hydroxybenzoyl)benzene and the mixture is reused.

## Revendications

1. Procédé de préparation du 1,4-bis-(4-hydroxybenzoyl)-benzène répondant à la formule I : 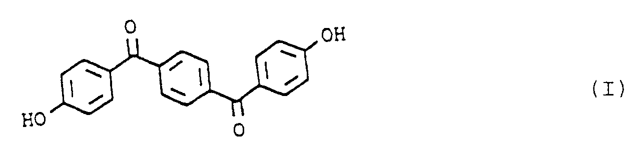 caractérisé en ce qu'on transpose le téréphtalate de diphényle répondant à la formule II : 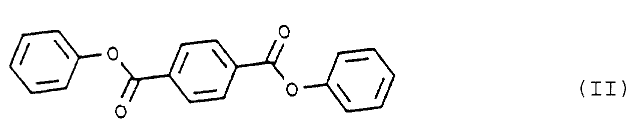 dans 3 à 50 parties d'un solvant organique anhydre inerte vis-à-vis des partenaires réactionnels, par rapport au téréphtalate de diphényle, en présence de 30 à 500 moles % d'un acide halogénoalcanesulfonique (catalyseur) répondant aux formules générales III ou IV :
Y(CₙX₂ₙ)SO₃H (III)
Y(CₙF₂ₙ)SO₃H (IV)
dans lesquelles Y représente un atome de fluor ou d'hydrogène, X un atome de fluor et/ou de chlore, sous réserve qu'au moins un X représente un atome de fluor et que n désigne un nombre entier de 1 à 10, à des températures de 10 à 200 °C.

2. Procédé selon la revendication 1, caractérisé en ce que l'on effectue la transposition à des températures d'environ 50 °C à environ 160 °C.

3. Procédé selon la revendication 1, caractérisé en ce que l'on effectue la transposition à des températures d'environ 80 °C à environ 130 °C.

4. Procédé selon au moins une des revendications 1 à 3, caractérisé en ce que Y, dans les formules générales III ou IV, désigne un atome d'hydrogène, et en ce que celui-ci est en position β par rapport au groupe -SO₃H.

5. Procédé selon au moins une des revendications 1 à 4, caractérisé en ce que l'on effectue la transposition en présence d'acide hexafluoropropanesulfonique ou d'acide trifluorométhanesulfonique.

6. Procédé selon au moins une des revendications 1 à 5, caractérisé en ce que l'on effectue la transposition dans du chlorobenzène, du dichlorobenzène, du chlorotoluène, du nitrobenzène, de la N-méthylpyrrolidone ou du sulfolan comme solvant organique inerte.

7. Procédé selon au moins une des revendications 1 à 6, caractérisé en ce que l'on effectue la transposition dans environ 5 à environ 20 parties du solvant organique inerte, par rapport au téréphtalate de diphényle.

8. Procédé selon au moins une des revendications 1 à 7, caractérisé en ce que l'on effectue la transposition du téréphtalate de diphényle dans le domaine de pressions allant de la pression atmosphérique à la pression spontanée du mélange réactionnel dans les conditions réactionnelles utilisées.

9. Procédé selon au moins une des revendications 1 à 8, caractérisé en ce que l'on effectue la transposition dans le domaine de pressions allant de la pression atmosphérique à environ 15 bars.

10. Procédé selon au moins une des revendications 1 à 9, caractérisé en ce que l'on effectue la transposition en l'absence d'oxygène.

11. Procédé selon au moins une des revendications 1 à 10, caractérisé en ce que l'on réutilise la liqueur-mère formée après séparation du 1,4-bis-(4-hydroxybenzoyl)-benzène.

12. Procédé selon au moins une des revendications 1 à 11, caractérisé en ce que l'on additionne le mélange réactionnel formé lorsque la transposition est terminée, après séparation du 1,4-bis-(4-hydroxybenzoyl)-benzène, de téréphtalate de diphényle et on le réutilise.

13. Procédé selon au moins une des revendications 1 à 10, caractérisé en ce que l'on effectue la transposition dans un procédé en un seul récipient, en partant de phénol et de dichlorure de l'acide téréphtalique, sans isolement intermédiaire du téréphtalate de diphényle, l'estérification préalable étant effectuée à des températures d'environ 10 à environ 200 °C.

14. Procédé selon la revendication 13, caractérisé en ce que l'on additionne le mélange réactionnel formé lorsque la transposition est terminée, après séparation du 1,4-bis-(4-hydroxybenzoyl)-benzène, de phénol/dichlorure de l'acide téréphtalique et on le réutilise.
